# EUROPEAN PATENT APPLICATION

(11) **EP 4 410 335 A1**
(43) Date of publication of application: **07.08.2024**
(21) Application number: 23154698.7
(22) Date of filing: 02.02.2023
(51) Int. Cl.: A61M 5/32

(54) **NEEDLE SAFETY DEVICE**

(71) Applicant: Sensile Medical AG, 4600 Olten (CH)
(72) Inventor: FORSTER, Richard, 92269 Fensterbach (DE); GORSHÖFER, Andreas, 93149 Nittenau (DE); JAKOB, Michael, 93051 Regensburg (DE); PFRANG, Jürgen, 93183 Kallmünz (DE)
(74) Representative: Bardehle Pagenberg Partnerschaft mbB Patentanwälte Rechtsanwälte

(57) **Abstract**

The present disclosure relates to a needle safety device (1) comprising an elongated hub portion (10) having a longitudinal axis (11). The hub portion (10) comprises a cannula (12) and a guide pin (13) extending outward from the hub portion (10). Further, the needle safety device (1) comprises a first tubular element (20) movably arranged on the hub portion (10) to move along the longitudinal axis (11). The first tubular element (20) comprises a guide track (21) slidably engaging with the guide pin (13). The guide track (21) is configured such that the first tubular element (20) rotates relative to the hub portion (10) when the guide pin (13) moves along the guide track (21). The guide track (21) has a final position (22) to immovably retain the guide pin (13). Further, the needle safety device (1) comprises a second tubular element (30) arranged on the first tubular element (20) such that it is movable along the longitudinal axis (11) with the first tubular element (20). The first tubular element (20) is at least partially enclosed by the second tubular element (30).

## Description

### 1. Technical field

The present disclosure relates to a needle safety device for a medical injection device and a medical injection device comprising the needle safety device.

### 2. Prior art

For some medical injection devices, such as auto-injectors or injection pens, it is often necessary to ensure that no intended and/or unintended reuse of the medical injection device can take place. The reasons for this can be manifold. For example, the medical injection device in question may be intended for only one injection procedure, such as an auto-injector that injects an emergency medication like adrenaline in the event of an allergic shock. In addition, it is understood that hygiene-related reasons are also conceivable. In this regard, especially cross-contamination after injection is to be avoided, where a person is injured and contaminated by a used needle.

Furthermore, for some medical injection devices, especially those being operated also by non-medical personnel, such as again auto-injectors or injection pens, it must be ensured that no unintentional contact with the cannula can take place. For example, to prevent injuries, contamination of the user, and/or contamination of the cannula.

Various solutions, in particular needle safety devices, are known from the prior art to prevent the reuse of a medical injection device and to prevent accidental contact with a cannula of the medical injection device. In general, known needle safety devices work on the principle that a sleeve is provided which is arranged around the needle in such a way that unintentional contact with this same needle is prevented. Further, the sleeve is configured to be displaced once by contact with an injection area such that injection can take place, whereas displacement of the sleeve again after injection is prevented, exemplarily by a locking means. However, the specific solutions of the prior art, respectively the existing needle safety devices, have several disadvantages, some of which are presented below.

First, some existing needle safety devices for medical injection devices cause the injection area to experience torsion. For example, because the sleeve surrounding the needle undergoes rotation relative to the medical injection device during injection, e.g. to effect locking. This rotation is regularly perceived by patients as unpleasant and, in the worst case, leads to the patient interrupting the injection.

Second, some needle safety devices allow a locking means, which is intended to fix the sleeve after injection in such a way that the cannula is permanently protected from contact, to be released again. One reason for this can be that the movement that led to the locking of the sleeve can be reversed manually, for example by turning the sleeve back to the unlocked initial state. Another reason can be that the locking means itself may be manually unlocked again. For example, by releasing a locking arm or, as in the embodiment shown in US 9,907,916 B2, by moving the pin out of the final position in which the pin is supposed to be retained.

Third, most needle safety devices have only one locking means to prevent the sleeve from returning to the unlocked initial position. Consequently, there is regularly no redundancy that would prevent the sleeve from returning to the unlocked initial position, for example if the one locking means fails or is deliberately disabled.

Thus, it is an object of the present disclosure to provide a needle safety device for a medical injection device and a medical injection device that overcome the aforementioned drawbacks at least partially.

### 3. Summary of the invention

This object is achieved, at least partly, by a needle safety device for a medical injection device and a medical injection device, as defined in the independent claims. Further aspects of the present disclosure are defined in the dependent claims.

In particular, the object is achieved by a needle safety device for a medical injection device. The needle safety device may serve to prevent the reuse of a medical injection device and to prevent accidental contact with a piercing means such as a cannula, wherein optionally the needle safety device may provide additional functionalities. The medical injection device maybe an auto-injector, an injection pen, and/or a syringe. However, preferably the medical injection device is an auto-injector. This is as auto-injectors particularly rely thereon that reuse is prevented and that accidental contact with the piercing means is avoided. Exemplarily since auto-injectors are regularly operated by non-medical personnel, e.g. in emergency situations.

The needle safety device comprises an elongated hub portion having a longitudinal axis, wherein the hub portion comprises a cannula extending from the hub portion substantially along the longitudinal axis and a guide pin extending outward from the hub portion as seen from the longitudinal axis. The longitudinal axis of the hub portion may also be a longitudinal center axis of the hub portion. Particularly, if the hub portion has a substantially cylindrical shape. The cannula may be also referred to as hollow needle or injection needle. Further, the cannula may be attached directly or indirectly to the hub portion. Said cannula may be part of a needle array. Moreover, it is understood that the cannula may be designed to be movable relative to the hub portion, for example to be able to realize a penetration of a liquid reservoir within the medical injection device. The guide pin may protrude outward from the hub portion as seen from the longitudinal axis substantially perpendicular to the longitudinal axis. Further, the guide pin may have a substantially cylindrical shape. However, the guide pin may alternatively comprise a substantially flat sliding surface, which preferably extends substantially perpendicular to the longitudinal axis. Advantages hereof are set out throughout the following.

Further, the needle safety device comprises a first tubular element being movably arranged on the hub portion to move along the longitudinal axis, wherein the first tubular element comprises a guide track which slidably engages with the guide pin, wherein the guide track is configured such that the first tubular element rotates relative to the hub portion when the guide pin moves along the guide track, wherein the guide track comprises a final position configured to retain the guide pin. Exemplarily, the first tubular element may be movably arranged on the hub portion by an inner surface of the first tubular element being slidably guided by an outside surface of the hub portion. The hub portion may be configured such that when the needle safety device is mounted to a medical injection device, it does not allow rotation relative to the medical injection device. Further, the aspect, wherein the final position is configured to retain the guide pin can mean that the guide pin is prevented from returning into other portions of the guide track. Hence, the needle safety device may thereby serve to prevent the reuse of a medical injection device. Furthermore, by means of the above-mentioned substantially flat sliding surface, which the guide pin may comprise, it can be achieved that in certain sections of the guide track, lower friction is realized than, for example, compared to a substantially cylindrical guide element. For example, by creating a planar contact between the guide pin and the guide track.

Moreover, the needle safety device comprises a second tubular element being arranged on the first tubular element such that the second tubular element is movable along the longitudinal axis together with the first tubular element, wherein the first tubular element is at least partially enclosed by the second tubular element, wherein the second tubular element comprises a contact surface for establishing pressure contact with an injection area. Exemplarily, the second tubular element may be arranged on the first tubular element such that an inner surface of the second tubular element is guided by an outside surface of the first tubular element. Moreover, the first tubular element may be enclosed by the second tubular element such that the guide pin and/or the guide pin are not accessible, e.g., by means of a human finger.

Furthermore, the needle safety device comprises a spring element which biases the first and/or second tubular element in a distal direction relative to the hub portion such that the contact surface is further spaced apart from the hub portion along the longitudinal axis than the tip of the cannula when the contact surface is not in pressure contact with the injection area. By means of the described biasing of the first and/or second tubular element an accidental contact with the tip of the cannula can be prevented. The spring element may be a coil spring. However, it is understood that different spring elements are conceivable.

The described needle safety device has several advantages over the prior art, wherein two of them are set out below in detail.

First, by the second tubular element being arranged on the first tubular element the injection area can be prevented from experiencing torsion. This is as the rotation of the first tubular element relative to the hub portion may be decoupled from the injection area by means of the second tubular element which comprises the contact surface. This can make the injection process more comfortable for a patient.

Second, by the first tubular element being at least partially enclosed by the second tubular element the needle safety device prevents the guide pin, when having reached the final position, to be released again. Particularly, the guide pin may not be manually unlocked again, i.e., moved out of the final position in which the guide pin is supposed to be retained. This is because intended and/or unintended contact with the guide pin and/or guide track can be avoided.

It is understood that the described advantages may apply also for the following in different expressions.

The guide track may comprise a first guide segment extending from an initial position to an injection position, wherein in the injection position the tip of the cannula is further spaced apart from the hub portion along the longitudinal axis than the contact surface, wherein the guide track is configured such that the guide pin moves into the injection position by the contact surface being in pressure contact with the injection area. The guide track may further comprise a second guide segment extending from the injection position to the final position, wherein the guide pin moves into the final position when the contact surface is released from pressure contact with the injection area. Hence, after injection, the cannula can be safely stored within the needle safety device. The first guide segment and the second guide segment may partially overlap.

Optionally, in the initial position and the final position the contact surface is further spaced apart from the hub portion along the longitudinal axis than the tip of the cannula. Hence, the second tubular element can prevent finger contact with the tip of the cannula, when the needle safety device is in the initial position or the final position. Exemplarily, when approaching the tip of the cannula with a human finger, the finger first abuts the contact surface instead of immediately contacting the tip of the cannula.

Further, the guide track may be configured such that the first tubular element rotates by a first angle around the longitudinal axis when the guide pin moves from the initial position to the injection position, and wherein the first tubular element rotates by a second angle around the longitudinal axis when the guide pin moves from the injection position to the final position.

The guide track may be configured such that the first tubular element rotates relative to the hub portion in one direction around the longitudinal axis while the guide pin moves from the initial position into the final position via the injection position. Hence, the first tubular element may rotate by a first angle around the longitudinal axis when the guide pin moves from the initial position to the injection position, and the first tubular element may rotate by a second angle in the same direction of rotation around the longitudinal axis when the guide pin moves from the injection position to the final position. For example, a short reversal of the direction of rotation need not be excluded. It has been shown that this configuration, specifically, prevents the first tubular element from being erroneously rotated back to its initial position after reaching the injection position.

Further, the contact surface may have an opening through which the tip of the cannula passes when the guide pin moves from the initial position into the injection position. Thereby the opening may have a diameter which avoids the insertion of a human finger into the second tubular element.

The first tubular element and the second tubular element may be configured such that they allow for a rotation relative to each other, wherein optionally the first tubular element is in rotatable sliding engagement with the second tubular element. Thus, the injection area can be particularly prevented from experiencing torsion. This is as the rotation of the first tubular element relative to the hub portion can be decoupled from the injection area by means of the second tubular element which comprises the contact surface. This makes the injection process more comfortable for a patient.

The second tubular element may be configured such that it substantially cannot rotate relative to the hub portion. This can ensure that the rotation of the first tubular element cannot be influenced from the outside of the needle safety device. Exemplarily, the second tubular element may directly engage with the hub portion. Alternatively, an intermediate element which is fixedly attached to the hub portion may ensure that second tubular element cannot rotate relative to the hub portion.

Moreover, the first tubular element and the second tubular element may comprise latching means being configured to allow the first tubular element to rotate relative to the second tubular element in one direction around the longitudinal axis and prevent rotation in the other direction. Hence, in addition to the final position of the guide track which retains the guide pin, an additional locking means to prevent the first tubular element from returning to the unlocked initial position is provided. Consequently, there is a redundancy that can prevent the first tubular element from returning to the unlocked initial position, for example if the engagement of the guide pin with the guide track in the final position fails to retain the guide pin. For example, the latching means may comprise ramps supported against each other, having undercuts so that after a relative movement of the ramps with respect to each other, they are locked and no reverse rotation can take place.

Optionally, the latching means prevents the guide pin to return into the initial position when it has been moved into the injection position and/or wherein the latching means prevents the guide pin from being moved out of the final position. This allows an additional locking means to be provided, particularly for the critical positions, thereby increasing the safety and/or reliability of the needle safety device.

The spring element may abut the hub portion and the second tubular element. This configuration has proven to be advantageous in that it prevents the spring element from affecting the rotation of the first tubular element.

Optionally, the first tubular element substantially cannot be displaced relative to the second tubular element along the longitudinal axis. This can reliably ensure that the guide track and guide pin remain inaccessible from the outside.

Furthermore, the hub portion may be substantially cylindrical. This allows for a particularly stable guiding of the first tubular element on the hub portion.

Further, the above object is achieved by a medical injection device comprising the needle safety device as described above. Since the medical injection device comprises the needle safety device as described above, it is understood that the features and/or advantages described with respect to the needle safety device may also apply for the medical injection device.

The medical injection device may comprise a housing which engages with the second tubular element such that the second tubular element substantially does not rotate relative to the hub portion, wherein optionally the hub portion is affixed to the housing.

### 4. Brief description of the accompanying figures

In the following, the accompanying figures are briefly described:
Fig. 1 shows in perspective view a needle safety device according to the present disclosure in the initial state;
Fig. 2 shows in perspective view the needle safety device during the injection process;
Fig. 3 shows in perspective view the needle safety device after finishing the injection process;
Fig. 4 shows a detail view of the guide track of the needle safety device;
Fig. 5 shows a cut view of the needle safety device in the initial state;
Fig. 6 shows a cut view of the needle safety device during the injection process;
Fig. 7 shows a perspective view of the first tubular element of the needle safety device;
Fig. 8 shows a perspective view of said first tubular element arranged on the hub portion of the needle safety device, and
Fig. 9 shows a perspective view of a medical injection device according to the present disclosure.

### 5. Detailed description of the figures

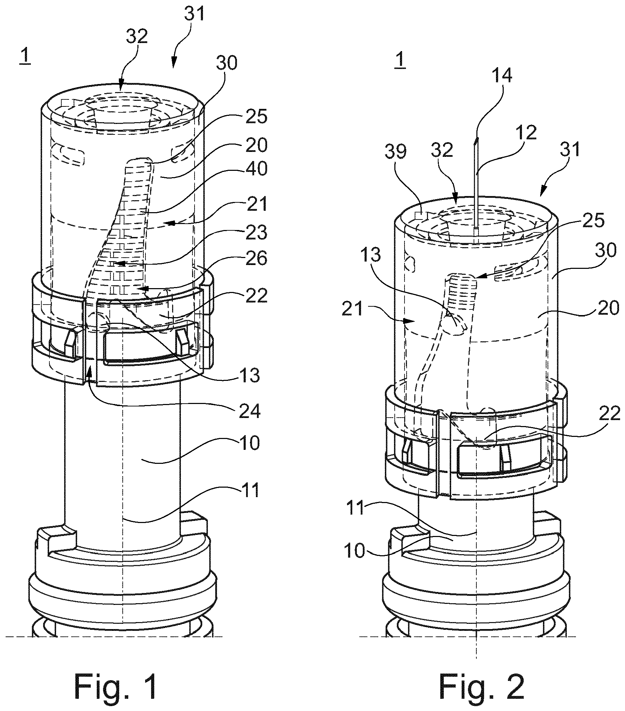

The **Figs. 1 to 9** all show the needle safety device 1 according to the present disclosure completely or part thereof.

As in **Fig. 1****,** the needle safety device 1 comprises an elongated hub portion 10 having a longitudinal axis 11. Since the hub portion 10 has a substantially cylindrical shape, it is understood that the longitudinal axis 11 of the hub portion 10 may also be referred to as a longitudinal center axis 11 of the hub portion 10.

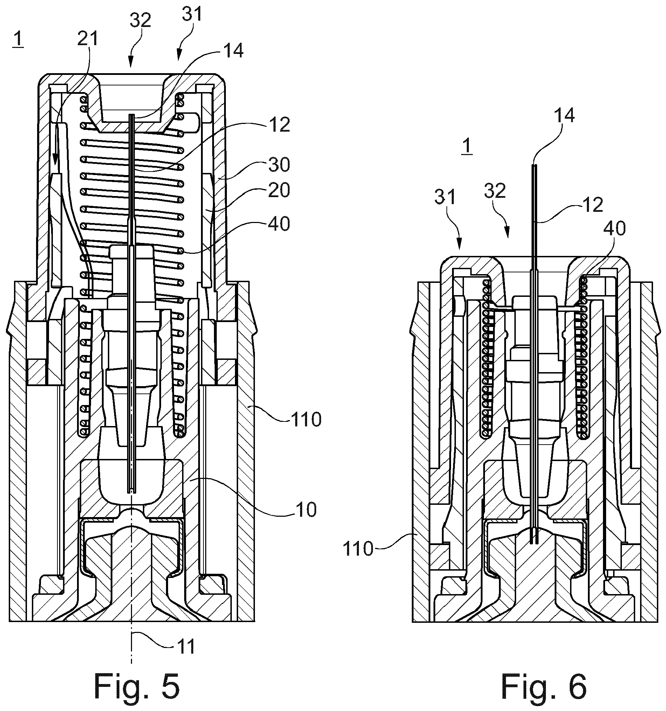

As particularly shown in **Figs. 5 and 6**, the hub portion 10 comprises a cannula 12 extending from the hub portion 10 along the longitudinal axis 11. The cannula 12 is attached indirectly to the hub portion 10 via a carrier portion that surrounds the cannula 12. Further, the cannula 12 is movable relative to the hub portion 10, to realize a penetration of a liquid reservoir within the medical injection device.

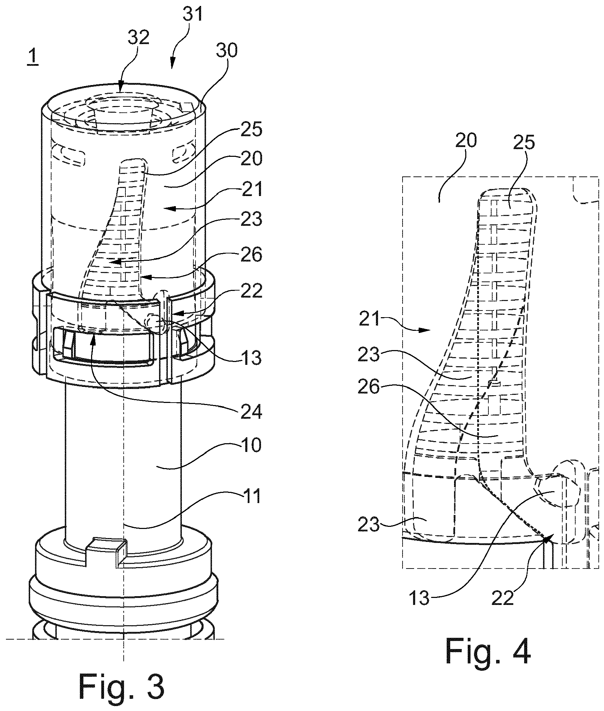

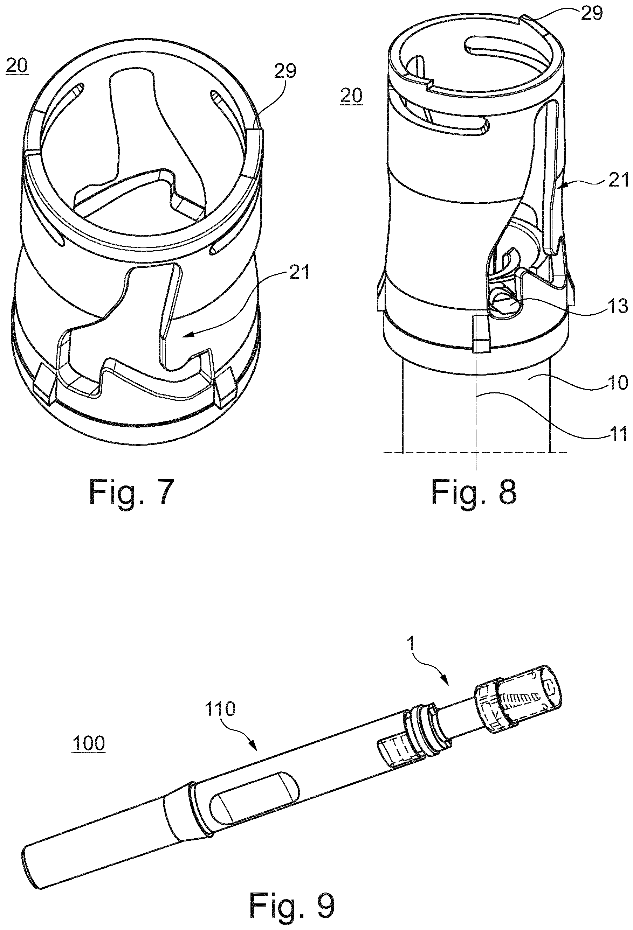

Moreover, as shown in detail in **Fig. 8**, the hub portion 10 further comprises a guide pin 13 extending outward from the hub portion 10 as seen from the longitudinal axis 11. Particularly, the guide pin 13 protrudes outward from the hub portion 10 as seen from the longitudinal axis 11 substantially perpendicular to the longitudinal axis 11. Further, as in **Fig. 4**, the guide pin 13 comprises a substantially flat sliding surface, which extends substantially perpendicular to the longitudinal axis 11.

The needle safety device 1 further comprises a first tubular element 20 being movably arranged on the hub portion 10 to move along the longitudinal axis 11. This is e.g., shown in specific detail in **Fig. 8****.** The first tubular element 20 comprises a guide track 21 which slidably engages with the guide pin 13. Said guide track 21 is configured such that the first tubular element 20 rotates relative to the hub portion 10 when the guide pin 13 moves along the guide track 21. The guide track 21 comprises a final position 22 configured to retain the guide pin 13. Exemplarily in **Figs. 3 and 4** the guide pin 13 has reached the final position 22. Thereby in **Fig. 4** is illustrated how the attempt to repeatedly uncover the cannula 12 is prevented. In detail, the guide pin 13 abuts a corresponding region of the guide track, thereby holding the guide pin 13 in the final position 22 so that the guide pin 13 is prevented from returning into other portions of the guide track 21.

By means of the substantially flat sliding surface, which the guide pin 13 comprises, it can be achieved that in certain sections of the guide track 21 a better sliding is realized than, for example, compared to a substantially cylindrical guide element. For example, by creating a planar contact between the guide pin and the guide track. This aspect will be understood by the skilled person when considering e.g., **Fig**. **4****.**

Moreover, the needle safety device 1 comprises a second tubular element 30 being arranged on the first tubular element 20 such that the second tubular element 30 is movable along the longitudinal axis 11 together with the first tubular element 20. The first tubular element 20 is enclosed by the second tubular element 30. Further, the second tubular element 30 comprises a contact surface 31 for establishing pressure contact with an injection area. Said contact surface 31 has an opening 32 through which the tip 14 of the cannula 12 passes when the guide pin 13 moves from the initial position 24 into the injection position 25.

Furthermore, the needle safety device 1 comprises a spring element 40 which biases the second tubular element 30 in a distal direction relative to the hub portion 10 such that the contact surface 31 is further spaced apart from the hub portion 10 along the longitudinal axis 11 than the tip 14 of the cannula 12 when the contact surface 31 is not in pressure contact with the injection area. Particularly, the spring element 40 being a coil spring abuts the hub portion 10 and the second tubular element 30.

As shown in **Figs. 1 to 4****,** the guide track 21 comprises a first guide segment 23 extending from an initial position 24 to an injection position 25. In said injection position 25 the tip 14 of the cannula 12 is further spaced apart from the hub portion 10 along the longitudinal axis 11 than the contact surface 31. The guide track 21 is configured such that the guide pin 13 moves into the injection position 25 by the contact surface 31 being in pressure contact with the injection area. Further, the guide track 21 comprises a second guide segment 26 extending from the injection position 25 to the final position 22, wherein the guide pin 13 moves into the final position 22 when the contact surface 31 is released from pressure contact with the injection area.

As depicted in **Fig. 1** **and** **3**, in the initial position 24 and the final position 22 the contact surface 31 is further spaced apart from the hub portion 10 along the longitudinal axis 11 than the tip 14 of the cannula 12. Hence, unintentional contact with the tip 14 of the cannula 12 is prevented.

Moreover, as illustrated by **Figs. 1 to 3**, the guide track 21 is further configured such that the first tubular element 20 rotates by a first angle around the longitudinal axis 11 when the guide pin 13 moves from the initial position 24 to the injection position 25, and wherein the first tubular element 20 rotates by a second angle around the longitudinal axis 11 when the guide pin 13 moves from the injection position 25 to the final position 22. Particularly, the guide track 21 is configured such that the first tubular element 20 rotates relative to the hub portion 10 in one direction around the longitudinal axis 11 while the guide pin 13 moves from the initial position 24 into the final position 22 via the injection position 25.

The first tubular element 20 and the second tubular element 30 are configured such that they allow for a rotation relative to each other, wherein the first tubular element 20 is in rotatable sliding engagement with the second tubular element 30. Particularly, as depicted in **Figs. 1 to 3**, protrusions of the first tubular element 20 slidingly engage with recesses of the second tubular element 30.

Moreover, even though not explicitly depicted, it is understood from **Figs. 1 to 3** that the second tubular element 30 is configured such that it cannot rotate relative to the hub portion 10. Exemplarily, this can be derived from the different positions of the protrusions of the first tubular element 20 within the recesses of the second tubular element 30, whereas the second tubular element 30 is not rotated. In detail, the housing 110, as shown in **Figs. 5 and 6****,** engages with the second tubular element 30 such that the second tubular element 30 substantially does not rotate relative to the hub portion 10.

Furthermore, as shown in **Figs. 2** **and** **8**, the first tubular element 20 and the second tubular element 30 comprise latching means 29, 39 being configured to allow the first tubular element 20 to rotate relative to the second tubular element 30 in one direction around the longitudinal axis 11 and prevent rotation in the other direction. Particularly, the latching means 29, 39 prevent the guide pin 13 to return into the initial position 24 when it has been moved into the injection position 25 and prevent the guide pin 13 from being moved out of the final position 22.

As the skilled person understands from **Figs. 1 to 3**, the first tubular element 20 substantially cannot be displaced relative to the second tubular element 30 along the longitudinal axis 11.

In **Fig. 9** the medical injection device 100 comprising the needle safety device 1 as described above is depicted. As shown, the hub portion 10 is affixed to the housing 110. However, different from **Figs. 5 and 6** the portion of the housing 110 which engages with the second tubular element 30 such that the second tubular element 30 substantially does not rotate relative to the hub portion 10 is not shown.

### List of reference signs

- 1: needle safety device
- 10: elongated hub portion
- 11: longitudinal axis
- 12: cannula
- 13: guide pin
- 14: tip of the cannula
- 20: first tubular element
- 21: guide track
- 22: final position
- 23: first guide segment
- 24: initial position
- 25: injection postion
- 26: second guide segment
- 29: latching means
- 30: second tubular element
- 31: contact surface
- 32: opening in the contact surface
- 39: latching means
- 40: spring element
- 100: medical injection device
- 110: housing

## Claims

1. A needle safety device (1) for a medical injection device (100), the needle safety device (1) comprising:
an elongated hub portion (10) having a longitudinal axis (11), wherein the hub portion (10) comprises a cannula (12) extending from the hub portion (10) substantially along the longitudinal axis (11) and a guide pin (13) extending outward from the hub portion (10) as seen from the longitudinal axis (11);
a first tubular element (20) being movably arranged on the hub portion (10) to move along the longitudinal axis (11), wherein the first tubular element (20) comprises a guide track (21) which slidably engages with the guide pin (13), wherein the guide track (21) is configured such that the first tubular element (20) rotates relative to the hub portion (10) when the guide pin (13) moves along the guide track (21), wherein the guide track (21) comprises a final position (22) configured to retain the guide pin (13);
a second tubular element (30) being arranged on the first tubular element (20) such that the second tubular element (30) is movable along the longitudinal axis (11) together with the first tubular element (20), wherein the first tubular element (20) is at least partially enclosed by the second tubular element (30), wherein the second tubular element (30) comprises a contact surface (31) for establishing pressure contact with an injection area, and
a spring element (40) which biases the first and/or second tubular element (20, 30) in a distal direction relative to the hub portion (10) such that the contact surface (31) is further spaced apart from the hub portion (10) along the longitudinal axis (11) than the tip (14) of the cannula (12) when the contact surface (31) is not in pressure contact with the injection area.

2. The needle safety device (1) according to the preceding claim, wherein the guide track (21) comprises
a first guide segment (23) extending from an initial position (24) to an injection position (25), wherein in the injection position (25) the tip (14) of the cannula (12) is further spaced apart from the hub portion (10) along the longitudinal axis (11) than the contact surface (31), wherein the guide track (21) is configured such that the guide pin (13) moves into the injection position (25) by the contact surface (31) being in pressure contact with the injection area, and
a second guide segment (26) extending from the injection position (25) to the final position (22), wherein the guide pin (13) moves into the final position (22) when the contact surface (31) is released from pressure contact with the injection area.

3. The needle safety device (1) according to the preceding claim, wherein in the initial position (24) and the final position (22) the contact surface (31) is further spaced apart from the hub portion (10) along the longitudinal axis (11) than the tip (14) of the cannula (12).

4. The needle safety device (1) according to any one of claims 2 to 3, wherein the guide track (21) is configured such that the first tubular element (20) rotates by a first angle around the longitudinal axis (11) when the guide pin (13) moves from the initial position (24) to the injection position (25), and wherein the first tubular element (20) rotates by a second angle around the longitudinal axis (11) when the guide pin (13) moves from the injection position (25) to the final position (22).

5. The needle safety device (1) according to any one of claims 2 to 4, wherein the guide track (21) is configured such that the first tubular element (20) rotates relative to the hub portion (10) in one direction around the longitudinal axis (11) while the guide pin (13) moves from the initial position (24) into the final position (22) via the injection position (25).

6. The needle safety device (1) according to any one of claims 2 to 5, wherein the contact surface (31) has an opening (32) through which the tip (14) of the cannula (12) passes when the guide pin (13) moves from the initial position (24) into the injection position (25).

7. The needle safety device (1) according to any one of the preceding claims, wherein the first tubular element (20) and the second tubular element (30) are configured such that they allow for a rotation relative to each other, wherein optionally the first tubular element (20) is in rotatable sliding engagement with the second tubular element (30).

8. The needle safety device (1) according to any one of the preceding claims, wherein the second tubular element (30) is configured such that it substantially cannot rotate relative to the hub portion (10).

9. The needle safety device (1) according to claims 7 and 8, wherein the first tubular element (20) and the second tubular element (30) comprise latching means (29, 39) being configured to allow the first tubular element (20) to rotate relative to the second tubular element (30) in one direction around the longitudinal axis (11) and prevent rotation in the other direction.

10. The needle safety device (1) according to claim 9 together with any one of claims 2 to 6, wherein the latching means (29, 39) prevents the guide pin (13) to return into the initial position (24) when it has been moved into the injection position (25) and/or wherein the latching means (29, 39) prevents the guide pin (13) from being moved out of the final position (22).

11. The needle safety device (1) according to any one of the preceding claims, wherein the spring element (40) abuts the hub portion (10) and the second tubular element (30).

12. The needle safety device (1) according to any one of the preceding claims, wherein the first tubular element (20) substantially cannot be displaced relative to the second tubular element (30) along the longitudinal axis (11).

13. The needle safety device (1) according to any one of the preceding claims, wherein the hub portion (10) is substantially cylindrical.

14. A medical injection device (100) comprising the needle safety device (1) according to any one of the preceding claims.

15. The medical injection device (100) according to the preceding claim, wherein the medical injection device (100) comprises a housing (110) which engages with the second tubular element (30) such that the second tubular element (30) substantially does not rotate relative to the hub portion (10), wherein optionally the hub portion (10) is affixed to the housing (110).
